# EUROPEAN PATENT SPECIFICATION

(11) **EP 4 126 169 B1**
(45) Date of publication and mention of the grant of the patent: **16.09.2026**
(21) Application number: 21774267.5
(22) Date of filing: 24.03.2021
(51) Int. Cl.: A61M 25/01, A61B 17/00, A61B 17/02, A61M 25/00, A61M 25/092, A61M 25/10

(54) **MEDIASTINUM ACCESS DEVICES**
MEDIASTINUM-ZUGANGSVORRICHTUNGEN
DISPOSITIF D'ACCÈS AU MÉDIASTINUM

(30) Priority: 24.03.2020 US 202062993955 P
(43) Date of publication of application: 08.02.2023
(73) Proprietor: Mayo Foundation for Medical Education and Research, Rochester, Minnesota 55905 (US)
(72) Inventor: ASIRVATHAM, Samuel J., Rochester, Minnesota 55906-8971 (US)
(74) Representative: Fish & Richardson P.C.
(86) International application number: PCT/US2021/023844
(87) International publication number: WO 2021/195184

(56) References cited:
- US-A1- 2004 167 558
- US-A1- 2005 228 452
- US-A1- 2008 306 333
- US-A1- 2009 076 498
- US-A1- 2015 223 757
- US-A1- 2015 230 699
- US-A1- 2015 313 634
- US-A1- 2016 213 462
- US-A1- 2017 304 624
- US-A1- 2018 098 850

## Description

### CROSS-REFERENCE TO RELATED APPLICATIONS

This application claims the benefit of U.S. Provisional Application Serial No. 62/993,955, filed March 24, 2020.

### BACKGROUND

### 1. Technical Field

This disclosure relates to devices and methods for accessing the mediastinum. For example, the disclosure relates to devices and methods for accessing the mediastinum in a minimally invasive manner without an intercostal incision and without deflating the lungs.

### 2. Background Information

The pericardium is the thin double-layered fluid filled sac that surrounds the heart and the roots of the aorta, vena cava, and the pulmonary artery. The outer sac is known as the fibrous pericardium. The inner sac is known as the serous pericardium. The serous pericardium consists of a visceral layer portion and a parietal layer portion ("parietal pericardium"). The visceral layer, or epicardium, covers the heart and the great vessels. The parietal portion lines the outer fibrous pericardium.

The mediastinum is the central compartment of the thoracic cavity surrounded by loose connective tissue, as an un-delineated region that contains a group of structures within the thorax. The mediastinum contains the heart and its vessels, the esophagus, the trachea, the phrenic and cardiac nerves, the thoracic duct, the thymus and the lymph nodes of the central chest.

US 2009/076498 A1 describes visualization and ablation system variations which utilize various tissue ablation arrangements. Such assemblies are configured to facilitate the application of bipolar energy delivery, such as RF ablation, to an underlying target tissue for treatment in a controlled manner while directly visualizing the tissue during the bipolar ablation process.

US 2016/213462 A1 describes systems and methods for performing transcatheter coronary artery bypass grafting procedures. The methods generally involve passing the graft from the aorta to the coronary artery through the pericardial space. The systems include poke-out wires, a coring device, and devices for forming anastomoses at the proximal and distal ends of a vascular graft.

US 2018/098850 A1 describes devices and methods for the treatment or repair of regurgitant cardiac valves, such as a mitral valve.

US 2015/313634 A1 describes a pericardial access device.

US 2005/228452 A1 describes steerable catheters.

### SUMMARY

The invention is defined by the features of the independent claim. Embodiments thereof are defined by the sub-features of the dependent claims.

This disclosure describes devices and methods for accessing the mediastinum without an intercostal incision and without deflating the lungs. In some embodiments, the devices and methods disclosed herein facilitate mediastinal access via a single percutaneous needle puncture. In some embodiments, two percutaneous needle punctures are used (e.g., a first puncture below the ribs or sternum, and a second puncture that is intercostal). Devices and methods to facilitate concurrent access to the pericardial and mediastinal spaces are also described herein. Multiple minimally invasive procedures are described that advantageously utilize the pericardial and mediastinal space access procedures described herein.

Without limitation, this disclosure describes exemplary devices and methods for at least the following subject matters: Entirely percutaneous methods, techniques, and devices to access the mediastinum and to treat a variety of diseases including, but not limited to, arrhythmia, asthma, pulmonary disorders, heart failure, syncope, hypertension, and others. This disclosure also describes exemplary devices and methods for percutaneous methods involving a subxiphoid transpericardial approach for coronary, bypass, and general Y-adaptation technique to create bypass shunts.

In one aspect, this disclosure is directed to a catheter device that includes a control mechanism, an elongate catheter shaft, a puncturing or cutting device, and an expandable member. The control mechanism is configured to be operated by a user exterior a patient. The elongate catheter shaft extends from the control mechanism and is configured to be positioned within the patient. One or more portions of the catheter shaft are controllably deflectable using the control mechanism to steer the catheter shaft. The puncturing or cutting device is attached to the catheter shaft and is configured to cut or puncture a parietal pericardial layer surrounding a heart of the patient. The expandable member is attached to a distal end portion of the catheter shaft. The expandable member is selectively expandable and collapsible using the control mechanism. The expandable member is configured to grasp a wire when the expandable member is reconfigured from an expanded configuration to a collapsed configuration.

Such a catheter device may optionally include one or more of the following features. The expandable member may be a metallic mesh with a shape-memory that biases the expandable member to seek the expanded configuration. The catheter device may also include one or more electrodes attached to the catheter device and configured for detecting electrical signals.

In another aspect, this disclosure is directed to a tissue reinforcement device. The tissue reinforcement device includes a first annular flange that is selectively reconfigurable between a contracted configuration and an expanded configuration, a second annular flange that is selectively reconfigurable between a contracted configuration and an expanded configuration, and a connection between the first and second annular flanges that defines an open lumen extending therebetween.

Such a tissue reinforcement device may optionally include one or more of the following features. The tissue reinforcement device may also include one or more sensors or electrodes attached to at least one of the first and second annular flanges. The tissue reinforcement device may be configured to be deployed in an opening of a parietal pericardial layer of a heart such that the first annular flange is outside of the heart and the second annular flange is inside a parietal cavity of the heart.

In another aspect, this disclosure is directed to a method that includes: (i) making a first percutaneous puncture and a first puncture of a parietal pericardium around a heart of a patient; (ii) advancing a puncture and expansion device through the first percutaneous puncture and the first puncture of the parietal pericardium such that a portion of the puncture and expansion device is located within a pericardial cavity of the heart; (iii) making a second puncture of the parietal pericardium using the puncture and expansion device while the puncture and expansion device is in the pericardial cavity; (iv) advancing the puncture and expansion device through the second puncture of the parietal pericardium and into a mediastinum of the patient; and (v) expanding an expandable portion of the puncture and expansion device while the expandable portion of the puncture and expansion device is in the mediastinum of the patient.

Such a method may optionally include one or more of the following features. The method may also include making a second percutaneous puncture and advancing a wire through the second percutaneous puncture so that a portion of the wire intersects with the expandable portion of the puncture and expansion device. The method may also include, while the wire intersects with the expandable portion of the puncture and expansion device, collapsing the expandable portion of the puncture and expansion device, wherein the expandable portion of the puncture and expansion device grasps the wire when collapsed. The method may also include pulling back the puncture and expansion device to cause the wire to pass through the pericardial cavity and exit the patient through the first percutaneous puncture.

Particular embodiments of the subject matter described in this document can be implemented to realize one or more of the following advantages. Devices and methods described herein can be used to access the mediastinum without a need to spread the ribs and without a need to artificially create pneumothorax. Complications such as persistent air leak are also avoided. Rather, mediastinal access can be accomplished through single needle access from the subxiphoid space, entering and exiting the pericardial space in an entirely percutaneous manner. In some embodiments, the mediastinum and pericardial cavity are concurrently accessed such that medical procedures can be advantageously performed in each. Many different types of health conditions can be treated in a minimally invasive fashion using the devices and methods provided herein. Such minimally invasive techniques can reduce recovery times, patient discomfort, and treatment costs.

Unless otherwise defined, all technical and scientific terms used herein have the same meaning as commonly understood by one of ordinary skill in the art to which this invention pertains. Although materials similar to those described herein can be used to practice the invention, suitable materials are described herein. In case of conflict, the present specification, including definitions, will control. In addition, the materials, methods, and examples are illustrative only and not intended to be limiting.

The details of one or more embodiments of the invention are set forth in the accompanying drawings and the description herein. Other features, objects, and advantages of the invention will be apparent from the description and drawings, and from the claims.

### DESCRIPTION OF THE DRAWINGS

FIG. 1 is a schematic diagram of patient undergoing a percutaneous pericardial sac puncture procedure using an exemplary catheter-based device in accordance with some embodiments provided herein. Here a distal end portion of the catheter-based device is protruding out from the pericardial sac and into the mediastinal space.
FIG. 2 shows the schematic diagram of FIG. 1 with an expandable mesh portion at the distal end portion of the catheter-based device in an expanded configuration in the mediastinal space.
FIGs. 3 and 4 are schematic diagrams of a heart and an example clamp device that can be installed on or around an opening of a pericardial sac to reinforce the pericardial tissue and provide an access portion through the parietal layer.
FIGs. 5A-D are a series of illustrations of a percutaneous coronary artery bypass graft surgery that can be performed using the devices and methods described herein.

Like reference numbers represent corresponding parts throughout.

### DETAILED DESCRIPTION

This disclosure describes devices and methods for accessing the mediastinum without an intercostal incision and without deflating the lungs. In some embodiments, the devices and methods disclosed herein facilitate mediastinal access via a single percutaneous needle puncture. This disclosure describes tools and techniques for accessing the pericardial cavity, and exiting the pericardial cavity through the parietal pericardium to get to the mediastinal space. In such a manner, the pericardial cavity is used as a pathway to get to the mediastinal space.

While in some embodiments the mediastinal space is accessed via the pericardial cavity using a single skin puncture, in some embodiments two percutaneous needle punctures are used (e.g., a first puncture below the ribs or sternum to access the pericardial cavity, and a second puncture that is intercostal). Moreover, multiple minimally invasive procedures are described herein that advantageously utilize the concurrent pericardial and mediastinal space access (e.g., embodiments related to phrenic nerve stimulation, percutaneous bypass surgery, as well as some iterations of cardiac assist devices and painless defibrillators as described below). That is, this document discloses a new method of percutaneous mediastinal access and in addition, specific inventions to utilize this new modality to treat a variety of disorders.

In accordance with some methods described herein, percutaneous access to the pericardial cavity is initially obtained in a standard manner. For example, to access the pericardial space non-surgically a needle can be insert under the sternum or rib cage. The needle can be advanced to puncture the parietal pericardium to reach the pericardial cavity. A wire can be advanced into the pericardial cavity via the needle.

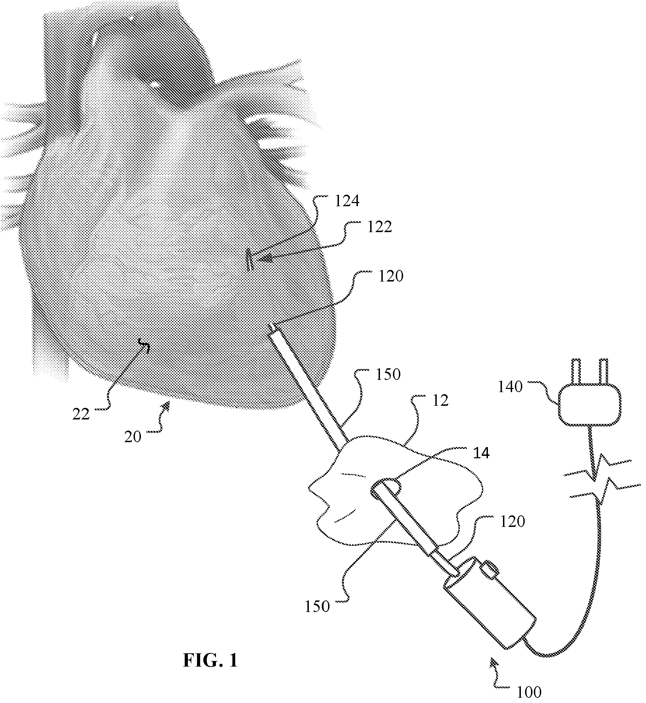

Once the pericardial cavity is entered, at least two specific devices as disclosed herein can be used. The first device, as depicted in FIG. 1, is a puncture and/or cutting tool with an expandable mesh that can puncture the parietal layer and then be advanced out of the pericardial cavity (by exiting through the parietal layer) and then positioned in the mediastinum. Within the mediastinum, the mesh can be expanded against the chest wall to displace a lung, for example. The mesh can also grasp onto and hold a wire when contracted.

With reference to FIG. 1, a human patient 10 is depicted as undergoing a procedure using an example percutaneous pericardial puncture and mediastinal retraction device 100 (or simply "device 100" for short). Patient 10 has a skin surface 12 with a skin penetration point 14, and a heart 20 that is encompassed by a pericardium 22.

Example device 100 includes a handle 110, a shaft 120, and an energy source connector 130. Dilator shaft 120 extends distally from handle 110. Connector 130 is coupled to handle 110 via a cable 132. Handle 110 includes an energy actuation switch 112. Dilator shaft 120 includes a distal tip portion 122 with an electrode 124.

In the depicted embodiment, example device 100 is a catheter-based device configured for percutaneous functionality. That is, device 100 is insertable through skin penetration point 14 (e.g., a puncture or an incision). The position of skin penetration point 14 can be, without limitation, in location such as, but not limited to the sub-xiphoidal, intercostal, and spaces such as the like.

In the depicted implementation, a delivery sheath 150 is positioned to direct the placement of device 100. In some cases, an endoscope is installed in patient 10 to direct the placement of device 100. In some cases, a trocar device is employed in skin penetration point 14. In some cases, a guidewire is used to direct the placement of device 100. In some embodiments, device 100 defines a lumen that can slidably receive a guidewire such that device 100 can be installed over the guidewire, or such that a guidewire can be installed using device 100 to direct the guidewire.

It should be understood that device 100 can be configured for other treatment techniques and modalities in addition to the depicted configuration. For example, device 100 can be configured for insertion into the vasculature of patient 10. In one such example configuration, device 100 is configured for percutaneous insertion into a femoral vein or internal jugular vein to attain transvenous access to the right atrium of heart 20. In such a case, device 100 can be used for puncturing the atrial septum of heart 20, for example. In another example, device 100 can be configured for puncturing into heart 20 (e.g., an atrial or ventricle puncture) to access a chamber of heart 20.

While device 100 is depicted as a single catheter, in some examples two or more catheter-based devices are used to perform the procedures provided herein. In some cases, multiple skin penetration points may be employed. Still further, in some examples, an open-chest procedure, or a thoracoscopy procedure can be used to perform the procedures provided herein.

In some embodiments, device 100 is configured to puncture tissue. For example, in the depicted embodiment device 100 is configured and arranged to puncture pericardium 22 to gain access to the pericardial space of heart 20.

Device 100 can include one or more structural features that configure it to puncture tissue. The distal tip portion 122 includes a pointed or sharpened distal tip portion. Moreover, in some such embodiments device 100 can be coupled to an energy source (e.g., a source of radio frequency (RF) energy) via a connector 130. In some examples, the energy supplied from such an energy source can be directed to or concentrated at one or more electrodes 124 at the distal tip portion 122. With energy from an energy source located at distal tip portion 122, distal tip portion 122 can more readily puncture a tissue such as the pericardium 22.

In some examples, a source of non-thermal energy is coupled to device 100 such that energy is delivered to and/or concentrated at distal tip portion 122. For example, in some examples direct current (DC) energy is supplied to device 100 such that the DC energy can be delivered from distal tip portion 122 to the tissue to be punctured. Such DC energy can facilitate electroporation of the cells of the tissue to promote a puncture of the tissue by distal tip portion 122.

In some embodiments, energy is concentrated at distal tip portion 122 without having electrodes located at distal tip portion 122. In some such examples, the device 100 includes a pointed distal tip portion 122 that naturally serves to concentrate the energy such that the energy has a higher charge density (electric field) at the pointed region of distal tip portion 122. The distal tip portion 122 has one or more electrodes and is additionally pointed to concentrate energy at the pointed tip.

In some examples, one or more portions of device 100 has dielectric properties. In some examples, the entire device 100 has dielectric properties. By exhibiting such dielectric properties, device 100 can be configured such that an electrical charge (AC or DC) can be concentrated at a pointed distal tip portion 122. In some examples, device 100 is made partially or entirely from one or more materials having dielectric properties such as, but not limited to, carbon, graphene, plastics, metal oxides, NuMetal, and the like, and combinations thereof. In some examples, a coating of one or more materials having dielectric properties is applied to device 100.

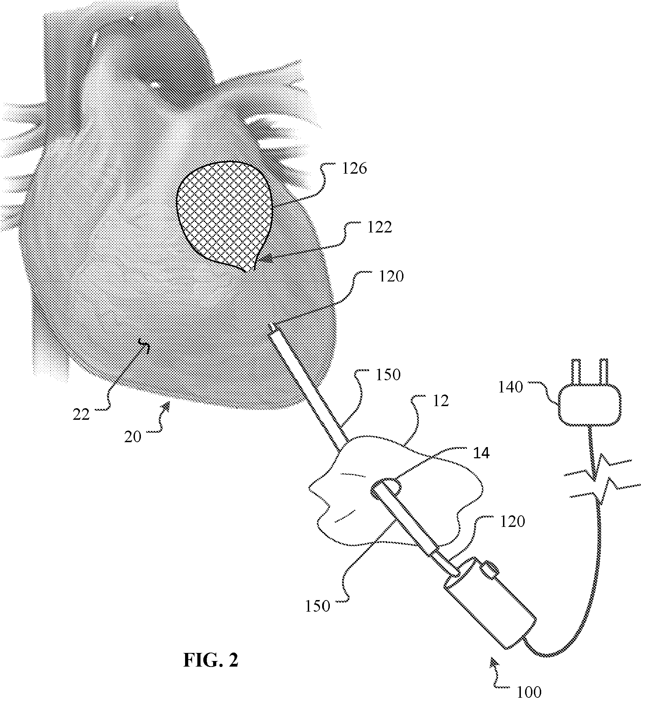

The puncture and expansion device 100 has multiple functionalities. First, as described above, it can puncture or cut through the parietal pericardial layer 22 as the distal tip portion 122 is entered into the pericardial space, and then again as the distal tip portion 122 is caused to exit the pericardial space and to enter into the mediastinal space. In addition, as shown in FIG. 2, the expandable portion can be expanded while in the mediastinal space to push the lungs out of the way (e.g., to provide retraction), for example.

The device 100 includes an expandable portion 126 at the distal tip portion 122 of the device 100. In some embodiments, the expandable portion 126 can be a metallic mesh or lattice (e.g., made from Nitinol or stainless steel) with shape memory. That is, in some embodiments the expandable portion 126 can have shape memory to enlarge to its expanded size/configuration when relieved from constraining forces. In some examples, the expandable portion 126 is or comprises a balloon.

The puncture and expansion device 100 is scalable to various suitable sizes (e.g., the expandable portion 126 can be 1 cm to 10 cm in diameter, and any size therebetween). In some examples, the puncture and expansion device 100 is steerable because one or more portions of the puncture and expansion device 100 are selectively deflectable. The expandable portion 126 can be selectively expandable and/or retractable *in situ.* When expanded, the expandable portion 126 can push the lungs and/or other organs or tissues out of the way. When collapsed, the expandable portion 126 can grab a wire that was pushed into the mesh of the expandable portion 126 while the mesh was in its expanded state.

In some procedures, the puncture and expansion device 100 can be advanced through the pericardial cavity and then positioned in the mediastinum adjacent the chest wall. Then, expandable portion 126 of the puncture and expansion device 100 can be expanded to displace one or both lungs (or other tissues and/or organs in the mediastinal space). Next, an intercostal puncture can be made through the chest adjacent to the expanded mesh of the expandable portion 126 (without concern about hitting a lung). Then, a wire can be percutaneously inserted via the intercostal puncture into the mesh of the expandable portion 126. Next, the mesh of the expandable portion 126 can be collapsed to attach onto the wire. Then the puncture and expansion device 100 can be pulled back so as to pull the wire into the pericardial cavity. Pulling back the puncture and expansion device 100 completely, and out through the puncture 14 of sub xiphoid, will create a wire loop within the patient's pericardial cavity and mediastinum, with both ends of the wire loop being outside of the patient. Such a wire loop can then be used in a very controllable manner for delivering other devices and therapies within the pericardial cavity, mediastinum, or both the pericardial cavity and mediastinum.

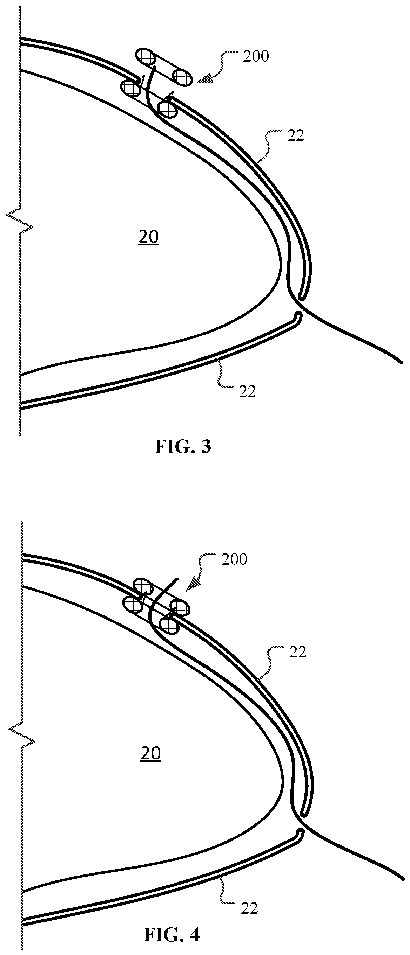

As depicted in FIGs. 3 and 4, a second device disclosed herein is a clasp or clamp device 200 (also referred to herein as "reinforcement device 200") that can be installed on the parietal layer of the pericardium 22 to provide an access port through the parietal layer to the mediastinum. The reinforcement device 200 can act as a port and as a mechanical stress-relief grommet to reinforce, stabilize, and/or secure the parietal layer. This reinforcement device will prevent unintentional tearing of the parietal layer as devices are passed through the parietal layer toward the mediastinum and vice versa.

In some examples, the reinforcement device 200 includes wire mesh flanges that can be deployed (one on each side of the parietal layer). In some examples, the reinforcement device 200 is constructed as a single piece, while in other examples the reinforcement device 200 is constructed of two or more pieces having a mechanical inter-connection like a latch (as shown). In some examples, the reinforcement device 200 is configured like a clamshell.

In some examples, the reinforcement device 200 can be used temporarily during the interventional procedure. Alternatively, in some examples the reinforcement device 200 can be left in the patient long-term. In some such examples, the reinforcement device 200 can include electrodes and/or sensors for monitoring cardiac and/or lung function. For example, the portion of the reinforcement device 200 exterior to the heart 20 is positioned to interact with the lungs (e.g., to sense lung function), while the portion of the reinforcement device 200 within the pericardial cavity is positioned to interact with the heart 20 (e.g., to electrically stimulate the heart 20).

The devices and methods described herein encompass a range of surgical devices and techniques. In some examples, the pericardial cavity and mediastinal access is performed percutaneously. The approach can be, for example, subxiphoidal or lateral or via thoracotomy. In some cases it may be possible to perform the procedure by entering the heart and puncturing out of the heart through a myocardial wall (e.g. ventricle, atrium, appendage, etc.) to gain access to the pericardial space (e.g., an inside-out approach). In some examples, video-assisted thoracoscopy can be used. In some examples, robotic assistance can be used. In some examples, open-chest techniques are used. In some examples, a combination of such techniques are used.

In some examples, the pericardial sac is initially punctured by a device, and then the cutting is performed from within the pericardial space in a direction generally outward from heart tissue.

Devices having a variety of functional features are used to perform the methods provided herein. In some examples, the devices provided herein can include flexible catheter-based grasping devices (e.g., forceps, suction devices, cryo/cooling devices, and/or snares). In some examples, the devices provided herein are flexible catheter-based cutting devices (e.g., scissors, sheaths, knife, scapel, lasers, snares, cryogenic devices, and electrocautery devices). In some examples, the grasping and cutting devices are combined on a single catheter device. In some examples, the grasping and cutting devices are separate devices. In some examples, the catheter-based devices provided herein can install hooks, anchors, and/or clips in a temporary manner to assist with performing the techniques. In some examples, an expandable element (e.g., a balloon, nitinol-based mesh devices, etc.) can be used to create a working space in the mediastinum and/or to protect the heart/lungs or otherwise direct the cutting instrument.

Components for visualizing, probing, and sensing the anatomy may be included with the devices provided herein. For example, in some examples the devices provided herein include electrode devices for stimulating or sensing nerves (e.g., the phrenic nerves). In some examples, the devices provided herein include an impedance measurement probe for sensing tissue or nerves. In some examples, the devices provided herein include a sonographic transducer such as a Doppler probe for visualizing the anatomy. In some examples, the devices provided herein include an optical camera for providing images of internal anatomy. In some examples, the devices provided herein include thoracoscopy style devices.

Other types of indirect visualization modalities may be used in conjunction with the devices and methods provided herein. For example, such indirect visualization modalities can include, but are not limited to, trans-esophageal echocardiography (TEE), magnetic resonance imaging (MRI), fluoroscopy (e.g., with use of injected contrast agents), computed tomography, thoracic echocardiography, tactile "imaging," and the like, and combinations thereof. Such visualization modalities can be utilized one or more of pre-procedurally, intra-procedurally, and/or post-procedurally.

Further additional functional features are included in some examples of devices provided for performing the methods provided herein. In some examples, stabilization devices are included. In some examples, balloon devices are included. In some examples, opposable elements with a coupling means are included. In some such examples, a first element can be located within the pericardial space, a second element can be located on the exterior of the pericardium, and a coupling between the two catheters can enable them to cooperatively perform a procedure.

The devices and methods described herein can be used to perform a number of new medical procedures, as follows.

### Placement of Defibrillators and Cardiac Pumps

Using the non-surgical access techniques described herein, the space in the mediastinum, adjacent to the pleura, can be used to contain a defibrillator that powers one or more electrodes positioned within the pericardial cavity. Such an arrangement provides an optimal vector for defibrillation operations (less energy to shock, less pain). In the case of such a defibrillator, the mediastinal component of the device acts as an electric shield, allowing relatively painless defibrillation.

More broadly, the mediastinum can be used accordingly as a vantage point to place various types of battery powered devices, capacitors, pumps, and other devices around the heart, with the option of having one or more parts in the pericardial space. In another example, an implantable heart pump (e.g., an LVAD) can be placed where the atria and aorta can be accessed within the pericardial space, but with the pump itself placed in the mediastinum.

### Asthma Treatment

The perihilar autonomic nerves are an important source for bronchospasm with asthma. With the approach described herein, a ring of electrodes can be deployed, e.g., either as a hook or a tied-ring around the hilum and/or related autonomic ridge centers. Simultaneously, using the techniques described herein, one or more electrode sensors (e.g., an anode and a cathode on mesh configured to electroporate or ablate) can be placed on the distal bronchi and in relation to the heart to monitor for, and respond to, untoward effects such as arrhythmia when stimulating or to test efficacy with airflow, etc., thus allowing for a feedback loop device.

### Emphysema Bullae and Lung Reduction Procedures

Using the new access system and technique as described herein, a diseased area of the lung, including bullae or emphysematous sites, can be lassoed or basket lassoed and thereby constricted with a ring of cauterizing electrodes. This can result in resected lung tissue that can be removed through the percutaneous access route. Cautery may be performed, e.g., using radiofrequency (RF) energy. Or, when combined as a treatment for bronchospastic disease, electroporation of the regional autonomic nerves in addition to cautery can be administered. Lung pumps can be implanted for patients with ventilatory failure in regions of the lung tissue. The lung pumps can be mechanical pumps with or without oxygenators, and with or without extra corporeal oxygenation, to effectively treat patients with end-stage lung disease.

### Examples for Sympathetic Denervation and Stimulation to Treat Hypertension and/or Syncope

The new devices and methods described herein can be used for treatment of hypertension, as well as syncope, with implanted devices. Presently, video-assisted thoracoscopic surgery ("VATS") procedures are performed to access the stellate ganglia and regional autonomics to treat hyperhidrosis and cardiac disturbances. Using the new devices and methods described herein, electrodes can be placed onto: (i) the stellate ganglia, (ii) in the pericardial cavity, and (iii) the nerves that are outside of the aorta. A stimulating device or blocking device placed in the mediastinum or near the abdomen can be coupled to the electrodes. In some examples, ring sensors for measuring blood pressure around the mediastinal portion of the aorta and electrode sensors can be placed in the pericardiac space and used with an indwelling device placed in the mediastinum adjacent to the pleura. When blood pressure falls the stellate and periaortic nerves are stimulated. When blood pressure rises, electroporation and blocking currents are used in similar locations to reduce the pressure.

The new devices and methods described herein, with the additional information provided by pericardial electrode, can be used to treat a greater number of arrhythmias with direct cardiac monitoring and the use of electroporation.

### Biopsy and Mediastinal Interventional Tools

The new devices and methods described herein can facilitate, with the aid of a mediastinoscope placed through the access but with the advantage that the lung is left in its entirety, and multiple accesses through the same site that can be utilized for biopsy, tumor removal, and other lung and mediastinal tissue removal procedures (including thymectomy) through the new access route as described herein.

In some examples, a pulsating pump device can be implanted, using the devices and methods described herein, to mechanically increase the blood pressure by squeezing areas such as the ventricles, the atria, the pulmonary arteries, etc. in accordance with the rhythm of the heart. Such a pump device is a temporary device that plays the roll of a heart/lung bypass pump machine.

### Vagus Nerve Stimulation and Blockade

The vagus nerve is readily accessed via the new access route as it courses around the esophagus. A small compressible circumesophageal ring electrode (e.g., clamp electrode) can be implanted at two separate sites to measure vagal activity and/or to stimulate or block vagal currents. Standard targets for vagal nerve stimulation (e.g., obesity, depression, epilepsy, etc.) can be affected with the advantage of real-time knowledge of whether the vagus was adequately stimulated and effects on esophageal and gastric motility enabled.

### Phrenic Nerve Stimulators

Another unique use of the devices and methods described herein is the placing of an electrode (e.g., clamp electrode) on the pericardial space side and on the mediastinal side of the phrenic nerve to encase the phrenic nerve for bipolar stimulation. There is no existing tool for such a scenario. Thus, the new non-surgical technique can be used to avoid extra phrenic stimulation and any risk of cardiac stimulation. Phrenic nerve stimulation has been shown to be of benefit in central sleep apnea, diaphragm paralysis, heart failure, and in some instances for a nonspecific treatment for dyspnea.

### Diaphragmatic and other Hernia Treatment

Diaphragmatic and hiatal hernias can be treated with this technique with an advantage over VATS procedures since valve-like devices and adjustable clamp devices can be anchored in the subxiphoid region for permanent treatment of hernias.

### Left and Right Ventricular Assist Devices

Through the mediastinum, the superior vena cava, pulmonary artery, and pulmonary veins are readily accessed using a Y-adapted shunt creation tool where structure A, such as the IVC or SVC is entered, structure B such as the pulmonary artery is entered. A phalange is then deployed, then a y-shaped tube advanced so that one limb enters structure A, one limb enters structure B, and a common limb used for the pump to take blood from one structure and into the other, for example, in the example sited, to function as a right ventricular assist device.

### Monitoring Devices

The mediastinal access thus obtained in a straightforward and easy manner will allow placement of a variety of unique monitoring tools with proximity to the great arteries, the major thoracic nerves, lungs, heart, and diaphragm.

### Edema Treatment Tool

Peripheral edema is usually from increased venous pressures in the SVC, IVC, and pulmonary veins. To perform this treatment, a device to create an inflatable ring so as to pulsate and thus load reduce these veins (in a manner akin to endovascular balloon assist devices) can be used to treat both peripheral and pulmonary edema.

### Pulmonary Hypertension Treatment

A unique use of the new devices and access technique described herein is the ability to access both the pericardial space and the extra pericardial mediastinal space. This dual access technique is critical for treating pulmonary hypertension with electroporation or other energy delivery since portions of the critical nerves on the pulmonary artery are positioned on either side of the pericardium. Electrodes will be positioned on the pulmonary artery on both sides of the pericardium. The electrodes of this device(s) will only fire their energy where neuro-signals from the pulmonary nerves are found (to avoid damaging the coronary arteries).

### Treatment of Gastroesophageal Reflux

To perform this treatment, a ring is advanced at the gastroesophageal junction around the diaphragm that along with stimulation can measure esophageal motility and stimulate or physically constrict appropriately to prevent reflux.

### Percutaneous Coronary Artery Bypass Graft Surgery

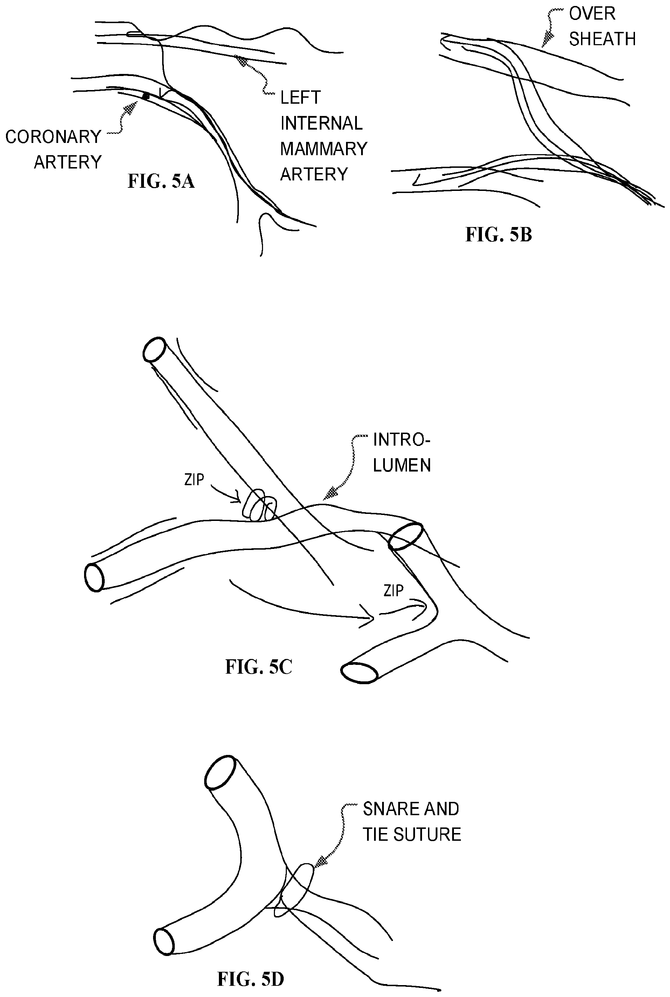

Referring to series of FIGs. 5A-D, percutaneous coronary artery bypass graft surgery can be performed using the devices and methods described herein. The left internal mammary artery is an extra pericardial structure that is readily visualized, and can be accessed through any thoracoscopic entry, but existing thoracoscopic techniques cannot effectively execute bypass surgery because of the lack of epicardial access where the epicardial coronary arteries are located.

With the use of the new devices and methods described herein, a pericardial port is used to visualize the proximal, mid, or distal coronary arteries, which are then entered with a limb of a y-shaped adapter. Once entered, the mediastinal port is used to similarly visualize and enter the left internal mammary artery (or the aorta) with the second limb of the y-shaped adapter. Once secure and the adjustable phalanges are in place to prevent bleeding, the common port is then positioned where blood will now flow from the site of higher pressure (such as the aorta or left internal mammary artery) to that of lower pressure such as the coronary arteries distal to the site of blockade in the coronary arteries.

Percutaneous access is obtained to the pericardial space with a cutting tool to exit the pericardium and expandable mesh closure devices to create a channel in the pericardium to allow placement of components in the pericardial space and in the extra pericardial mediastinal space. In some examples, a single deflectable catheter is mounted with a pericardial/thoracic scope and utilized to deliver wires and visualize the coronary vessels and the internal thoracic vessels as well as aorta.

A Y-adaptation tool can be used for shunt creation. After appropriate visualization, vessel A is entered with a wire. Vessel B is entered separately through another wire. Vessels A and B are to be y-adapted to create a shunt.

Once the vessel has been entered, a closure device with a lumen is advanced to either side of both vessel walls and left in place, maintaining wire access. Through this, a lumen with an end hole and a proximal side hole tube is advanced into vessel A such that the end hole points downstream in vessel A. Through the side hole, the wire that had been advanced to vessel B is now placed through the lumen so as to have proximal access. Through this wire, a second lumened tube is advanced to vessel B and secured with a similar clasping mesh lock as used above for initial access. Now, through the wire that went out through the side hole of the initial lumened tube through vessel A, a clasping mesh or other lock is placed so that the two vessels are now connected with vessel B's end lumen pointing upstream to blood flow.

The redundant portion of the second lumen which is internal to the first lumen is now cut with a lasso-like snare and removed. Pledgets are then advanced towards the connecting segment between the two vessels and now the main tube is cut with a snare and the shunt left in place.

In some examples, the proximal limb of the Y has a zipper-like device that can be fastened externally through a percutaneous catheter so as to create a more obtuse angle to the Y, promoting blood flow from one vessel to another.

In some examples, a catheter mounted percutaneous "pushing" device with a reverse axilla is used to push and evert the connecting limb of the Y to once again create a near 180 degree angle between vessel A and vessel B.

The inventive devices as described above involve vessel A being the distal part of a coronary artery that has been occluded with atherosclerotic disease and vessel B the left internal mammary artery. However, it is also envisioned that vessel A and vessel B may be proximal and distal to any obstructing segment, and the technique can be used to create shunts for veins, large arteries, vessels, chambers of the heart, neurological shunts, gastrointestinal shunts, and other areas where shunting of one lumen structure to another is necessary.

### Additional or Alternative Features/Examples

Although devices and methods described above allow the performance of a vats-like procedure without violating the lung, entering the pleural space is not precluded and in fact, with some iterations, we purposely enter the pleural space either after exiting the pericardium or at a site where there is pleural pericardial adherence (pleural membranes and parietal pericardium are together). This allows access on the outside surface of the lung without necessarily damaging and then, if so desired, re-exiting from the pleural space to get to the mediastinum. This important aspect allows free mobility of either someone manually manipulating the devices or using a robot to get to multiple locations like the sympathetic chain, diaphragmatic structures, etc. In other words, this disclosure provides devices and methods for transpericardial exit and subsequent intraparietal pleura entrance (e.g., entrance of the pleural space via the pericardial space). With such access to the pleural space gained, treatments such as electroporation of the surface of the lung, placement of implanted devices in the pleural space, and other medical treatments can be delivered.

It should be understood that the scope of the instant disclosure includes placing monitoring devices for lung pressures, heart pressures, pericardial pressure, mediastinal pressures, tidal volume measures, valve function, heart function, etc., in the mediastinal space, pericardial space, and pleural space using the techniques described herein. Similarly, all of these approaches and vantage points could be used to deliver drugs, biologicals, stem cells, etc.

Although a unique aspect of this innovation is having the ability to attach an extra pericardial vessel like the left internal mammary to an interpericardial vessel like the left anterior descending artery, the same tools and techniques for approximation and conduit creation would be to bypass an obstruction from one portion of the intrapericardial vessel to another or from the aorta to an intrapericardial epicardial coronary artery.

It should be understood that the scope of the instant disclosure includes placing fluorescent and other dyes in the pericardial space to better visualize the coronary arteries and using a pericardial scope to identify where the coronary arteries are, and then using the same scope to identify the left internal mammary artery. For the pacing lead or stimulatory electrodes that will be placed in ganglia like the stellate ganglia or other ganglia, the lead itself may contain electrodes or sensors that when implanted can continuously monitor cardiac, vascular, and other thoracic-based physiological parameters.

The devices and methods described herein can also be used for the atrium including all the various sensing algorithms, delivery sequences, etc. If a patient has an implanted defibrillator already, the existing coil may be adapted and used for electroporation delivery with a novel pulse generator, or the defibrillator coil may be one limb (one element of the electroporation field) while novel electrodes may be used as the other limb.

While this specification contains many specific implementation details, these should not be construed as limitations, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described herein should not be understood as requiring such separation in all embodiments, and it should be understood that the described program components and systems can generally be integrated together in a single product or packaged into multiple products.

Particular embodiments of the subject matter have been described. Other embodiments are within the scope of the following claims. As one example, the processes depicted in the accompanying figures do not necessarily require the particular order shown, or sequential order, to achieve desirable results. In certain implementations, multitasking and parallel processing may be advantageous.

## Claims

1. A catheter device (100) comprising:
a control mechanism (110) configured to be operated by a user exterior a patient (10);
an elongate catheter shaft (120) extending from the control mechanism (110) and configured to be positioned within the patient, wherein one or more portions of the catheter shaft (120) are controllably deflectable using the control mechanism (110) to steer the catheter shaft (120);
a puncturing device attached to the catheter shaft (120), the puncturing device configured to puncture a parietal pericardial layer surrounding a heart (20) of the patient (10), and
an expandable member (126) attached to a distal end portion of the catheter shaft (120), the expandable member (126) being selectively expandable and collapsible using the control mechanism (110),
wherein the expandable member (126) is configured to grasp a wire when the expandable member (126) is reconfigured from an expanded configuration to a collapsed configuration, **characterised in that** the puncturing device comprises a pointed distal tip portion (122), and **in that** the pointed distal tip portion (122) comprises multiple electrodes (124).

2. The catheter device of claim 1, wherein the expandable member is a metallic mesh with a shape-memory that biases the expandable member to seek the expanded configuration.

3. The catheter device of claims 1 or 2, further comprising one or more electrodes attached to the catheter device and configured for detecting electrical signals.

## Patentansprüche

1. Kathetervorrichtung (100), umfassend:
einen Steuermechanismus (110), der dazu konfiguriert ist, von einem Benutzer außerhalb eines Patienten (10) bedient zu werden;
einen länglichen Katheterschaft (120), der sich von dem Steuermechanismus (110) erstreckt und dazu konfiguriert ist, innerhalb des Patienten positioniert zu werden, wobei ein oder mehrere Abschnitte des Katheterschafts (120) unter Verwendung des Steuermechanismus (110) steuerbar auslenkbar sind, um den Katheterschaft (120) zu lenken;
eine Punktionsvorrichtung, die an dem Katheterschaft (120) angebracht ist, wobei die Punktionsvorrichtung dazu konfiguriert ist, um eine parietale perikardiale Schicht, die ein Herz (20) des Patienten (10) umgibt, zu punktieren,
und
ein expandierbares Element (126), das an einem distalen Endabschnitt des Katheterschafts (120) angebracht ist, wobei das expandierbare Element (126) unter Verwendung des Steuermechanismus (110) selektiv expandierbar und kollabierbar ist,
wobei das expandierbare Element (126) dazu konfiguriert ist, einen Draht zu ergreifen, wenn das expandierbare Element (126) von einer expandierten Konfiguration in eine kollabierte Konfiguration umkonfiguriert wird, **dadurch gekennzeichnet, dass** die Punktionsvorrichtung einen spitzen distalen Spitzenabschnitt (122) umfasst, und dass der spitze distale Spitzenabschnitt (122) mehrere Elektroden (124) umfasst.

2. Kathetervorrichtung nach Anspruch 1, wobei das expandierbare Element ein metallisches Maschenmaterial mit einem Formgedächtnis ist, welches das expandierbare Element vorspannt, um die expandierte Konfiguration anzustreben.

3. Kathetervorrichtung nach Anspruch 1 oder 2, ferner umfassend eine oder mehrere Elektroden, die an der Kathetervorrichtung angebracht und zum Detektieren elektrischer Signale konfiguriert sind.

## Revendications

1. Dispositif de cathéter (100) comprenant :
un mécanisme de commande (110) configuré pour être actionné par un utilisateur extérieur à un patient (10) ;
une tige allongée de cathéter (120) s'étendant à partir du mécanisme de commande (110) et configurée pour être positionnée à l'intérieur du patient, une ou plusieurs parties de la tige de cathéter (120) pouvant être fléchies de manière contrôlable à l'aide du mécanisme de commande (110) pour diriger la tige de cathéter (120) ;
un dispositif de perforation fixé à la tige du cathéter (120), le dispositif de perforation étant configuré pour perforer une couche péricardique pariétale entourant un cœur (20) du patient (10),
et
un élément expansible (126) fixé à une partie d'extrémité distale de la tige de cathéter (120), l'élément expansible (126) étant sélectivement expansible et repliable à l'aide du mécanisme de commande (110),
l'élément expansible (126) étant configuré pour saisir un fil lorsque l'élément expansible (126) est reconfiguré d'une configuration expansée à une configuration repliée, **caractérisé en ce que** le dispositif de perforation comprend une partie de pointe distale (122), et **en ce que** la partie de pointe distale (122) comprend de multiples électrodes (124).

2. Dispositif de cathéter selon la revendication 1, l'élément expansible étant un treillis métallique avec une mémoire de forme qui sollicite l'élément expansible pour rechercher la configuration expansée.

3. Dispositif de cathéter selon la revendication 1 ou 2, comprenant en outre une ou plusieurs électrodes fixées au dispositif de cathéter et configurées pour la détection de signaux électriques.
